# FASCICULE DE BREVET EUROPEEN

(11) **EP 3 119 537 B1**
(45) Date de publication et mention de la délivrance du brevet: **13.02.2019**
(21) Numéro de dépôt: 15714944.4
(22) Date de dépôt: 16.03.2015
(51) Int. Cl.: B09B 3/00, C02F 11/04, C05F 17/00, C12M 1/107, C12M 1/33

(54) **PROCEDE DE TRAITEMENT DE DECHETS, EN PARTICULIER D'ORDURES MENAGERES, ET INSTALLATION POUR LA MISE EN OEUVRE DU PROCEDE**
VERFAHREN ZUR BEHANDLUNG VON ABFÄLLEN, INSBESONDERE HAUSMÜLL, SOWIE ANLAGE ZUR DURCHFÜHRUNG DES VERFAHRENS
PROCESS FOR TREATING WASTE, IN PARTICULAR HOUSEHOLD REFUSE, AND FACILITY FOR THE IMPLEMENTATION OF THE PROCESS

(30) Priorité: 18.03.2014 FR 1452208
(43) Date de publication de la demande: 25.01.2017
(73) Titulaire: SUEZ Groupe, 92040 Paris la Défense Cedex (FR); Vinci Environnement, 92500 Rueil Malmaison (FR)
(72) Inventeur: FLEURY, Sylvie, F-78125 Orphin (FR); SOMMAIN, Arnaud, F-34110 Mireval (FR); GREZE, Olivier, F-34970 Lattes (FR); THIBAUT, Romain, F-78360 Montesson (FR); SEUTIN, Hugues, F-78150 Le Chesnay (FR)
(74) Mandataire: IPAZ
(86) Numéro de dépôt international: PCT/IB2015/051909
(87) Numéro de publication internationale: WO 2015/140692

(56) Documents cités:
- EP-A2- 2 364 782
- FR-A- 1 091 088
- US-A1- 2009 095 673

## Description

L'invention est relative à un procédé de traitement de déchets, en particulier d'ordures ménagères, contenant des matières organiques mélangées à des inertes, notamment métaux, matières minérales, plastiques, verre, procédé du genre de ceux selon lesquels :
- les déchets sont soumis à un premier criblage,
- la fraction des déchets ayant traversé ce premier criblage est soumise à un traitement de pré-fermentation dans un tube rotatif avec alimentation à une extrémité et extraction à l'autre extrémité,
- les déchets, après traitement de pré-fermentation et, le cas échéant après criblage intermédiaire, sont soumis à une étape de criblage fin pour ne laisser passer qu'une fraction de matières de taille inférieure ou égale à une dimension M, en particulier inférieure ou égale à 20 mm, correspondant essentiellement à des matières organiques, et pour donner un refus de criblage fin.

Les déchets ménagers issus de collectes non sélectives contiennent différentes catégories de matériaux telles que des déchets putrescibles (déchets alimentaires, déchets verts), des papiers, des cartons, du verre, des plastiques, des métaux ferreux ou non ferreux, des tissus, des textiles sanitaires, des produits toxiques (piles électriques, pots de peinture...).

La méthanisation ou le compostage de ces déchets ménagers en vue de les transformer en biogaz ou en compost valorisable comporte généralement quatre étapes principales :
- une préparation mécanique des déchets qui vise à séparer les matières organiques biodégradables des autres fractions non valorisables en biogaz ou en compost ; un tube rotatif de pré-fermentation, généralement horizontal, constitue un moyen pour une telle préparation ;
- la méthanisation qui vise à produire une énergie renouvelable et est réalisée dans des enceintes horizontales ou verticales, agitées mécaniquement ou par brassage au biogaz, éventuellement par une boucle de recirculation;
- le compostage, ou maturation aérobie, qui fait généralement intervenir une opération préalable de pressage ou de déshydratation d'un digestat provenant de la méthanisation, afin d'atteindre un niveau de teneur en matières sèches et une porosité permettant l'auto-compostage du digestat, ou alors nécessite l'apport d'un agent structurant et son mélange avec le digestat pour obtenir un substrat compostable ;
- un affinage final qui vise à retirer de façon complémentaire les contaminants qui subsistent après les deux opérations précédentes, et à préparer le compost à une granulométrie permettant sa valorisation agronomique.

Le traitement des déchets nécessite donc un tri qui permettra notamment de séparer les matières organiques biodégradables des autres fractions non valorisables en biogaz ou en compost.

Le domaine technique de l'invention se situe dans le contexte particulier de l'étape de préparation mécanique des déchets en vue d'une méthanisation ou d'un compostage ultérieur.

Un criblage fin, utilisant une maille fine de taille inférieure ou égale à M, en particulier inférieure ou égale à 20 mm, permet d'optimiser la collecte des matières organiques qui pourront être valorisées.

Un procédé de traitement des déchets de ce genre est connu notamment d'après FR 2 988 624 et FR 2 988 625. Autres procédés de traitement des déchets sont connus d'après US 2009/095673 A1, FR 1 091 088 A et EP 2 364 782 A2.

Les cribles à mailles fines, en particulier inférieures à 10 mm, et même inférieures 8 mm, permettent de limiter la présence d'inertes en filière biologique de type valorisation, en compost ou en méthanisation. Toutefois, un inconvénient d'un tel criblage fin réside dans la perte des matières organiques qui restent dans les fractions supérieures au maillage et qui se trouvent dans le refus du criblage fin.

Actuellement, les fractions grossières, correspondant à des refus de criblage, sont traitées en méthanisation/compostage, avec un affinage en fin de traitement. Certaines filières prévoient un affinage en tête et destinent les fractions plus grossières à des filières telles que stabilisation ou méthanisation en vue d'une valorisation énergétique optimisée ou d'une utilisation comme combustible de substitution. Le taux de refus d'un tel procédé, c'est-à-dire le rapport de la masse de déchets refusée par le criblage à la masse totale, se situe entre environ 45 % et 60 %, avec un faible taux de compost produit conformément aux normes.

Ce taux de refus est en général largement supérieur à la proportion en masse de matières organiques présentes dans les déchets. Le bilan matières d'un tel traitement mécanique/biologique est fortement dégradé au vu de la quantité de matières organiques présentes dans les refus et non mobilisables pour un compost de qualité.

Les inertes sont effectivement de plus en plus réduits en serrant le maillage des cribles, mais les quantités de matières organiques récupérées sont aussi réduites.

L'invention a pour but, surtout, de mieux mobiliser, c'est-à-dire mieux récupérer, la matière organique présente dans la fraction des matières soumise à l'étape de criblage fin, cette fraction de matières correspondant à des dimensions généralement inférieures à 80 mm, avantageusement inférieures à 30 mm, et encore plus avantageusement inférieures à 20 mm.

Selon l'invention, un procédé de traitement de déchets du genre défini précédemment, est caractérisé en ce que :
- le refus de criblage fin est soumis à un traitement mécanique et/ou biologique et/ou chimique pour la mobilisation de la matière organique, ce traitement étant suffisant pour fragmenter la matière organique présente dans le refus de criblage fin tout en assurant un maintien de la taille des inertes et en évitant leur fragmentation,
- et le refus de criblage fin, après ce traitement, est recirculé vers l'étape de criblage fin.

De préférence, une étape de déconcentration/séparation des inertes est prévue entre la sortie du refus de criblage fin et l'entrée du traitement de mobilisation, pour écarter les inertes de la fraction organique. Cette étape de déconcentration/séparation peut comprendre un tri balistique / densimétrique, et / ou un tri optique et / ou une séparation gravitaire sur tapis roulant incliné.

Avantageusement, le criblage fin est effectué avec un maillage dont la dimension M est comprise entre 5 et 10 mm, tandis que les déchets soumis à ce criblage fin sont issus d'un crible situé en amont dont la maille est de 20 mm.

Le traitement de mobilisation de matières organiques de la fraction refusée par le criblage fin se situe entre 5 et 20 mm, et le traitement de mobilisation permet de transférer la matière organique vers la fraction 0-5 mm.

Le traitement de mobilisation de matières organiques peut comprendre une étape de stockage dans un dispositif de stockage aéré et brassé, en particulier une fosse, pendant une durée de quelques heures à plusieurs jours, en particulier de 24 h.

Selon une autre possibilité, le traitement de mobilisation des matières organiques peut comprendre le passage des matières dans un tube rotatif, de préférence aéré avec de l'air à température ambiante ou réchauffé, équipé de moyens propres à fragmenter la matière organique, sans réduire la taille des inertes, en particulier de boulets ou de couteaux.

L'invention est également relative à une installation pour la mise en oeuvre du procédé défini précédemment, cette installation comprenant :
- un premier crible pour les déchets,
- un tube rotatif avec alimentation à une extrémité par la fraction de déchets ayant traversé le premier crible, et extraction à l'autre extrémité,
- des cribles intermédiaires éventuels,
- et un crible fin pour laisser passer une fraction de matières de taille inférieure ou égale à une dimension M, en particulier inférieure ou égale à 20 mm,
et étant caractérisée en ce qu'elle comporte :
- un dispositif à action mécanique et/ou biologique et/ou chimique de mobilisation des matières organiques du refus de criblage fin, propre à fragmenter la matière organique présente dans le refus tout en assurant le maintien de la taille des inertes,
- et une boucle de recirculation du refus de criblage fin avec passage par le dispositif de mobilisation de matières organiques.

Avantageusement, l'installation comporte un équipement de déconcentration/séparation des inertes prévu entre la sortie du refus de criblage fin et l'entrée du dispositif de mobilisation, pour écarter les inertes de la fraction organique. L'équipement de déconcentration/séparation peut comprendre un tri balistique / densimétrique, et / ou un tri optique et / ou une séparation gravitaire sur tapis roulant incliné.

De préférence, le criblage fin est effectué avec un maillage dont la dimension M est égale à 5 mm, tandis que les déchets soumis à ce criblage fin sont issus d'un crible situé en amont dont la maille est de 20 mm.

Le dispositif de mobilisation des matières organiques peut comprendre un dispositif de stockage aéré et brassé, en particulier une fosse avec plancher muni d'ouvertures d'aération.

Selon une autre possibilité, le dispositif de mobilisation des matières organiques peut comprendre un tube rotatif, équipé de moyens propres à fragmenter la matière organique, sans réduire la taille des inertes, en particulier de boulets ou de couteaux.

L'invention consiste, mises à part les dispositions exposées ci-dessus, en un certain nombre d'autres dispositions dont il sera plus explicitement question ci-après à propos d'exemples de réalisation décrits avec référence aux dessins annexés, mais qui ne sont nullement limitatifs. Sur ces dessins :
Fig. 1 est un schéma synoptique partiel illustrant un procédé de traitement selon l'invention.
Fig. 2 est une vue schématique, à plus grande échelle que sur Fig. 1, du crible fin et de la boucle de recirculation avec dispositif de mobilisation de la matière organique.
Fig. 3 est une vue schématique en élévation, à plus petite échelle, d'un tambour rotatif pour le traitement de mobilisation de matières organiques.
Fig. 4 est une vue de gauche par rapport à Fig. 3 du tambour et
Fig. 5 est une vue en perspective schématique d'une variante de tambour pour le traitement de mobilisation.

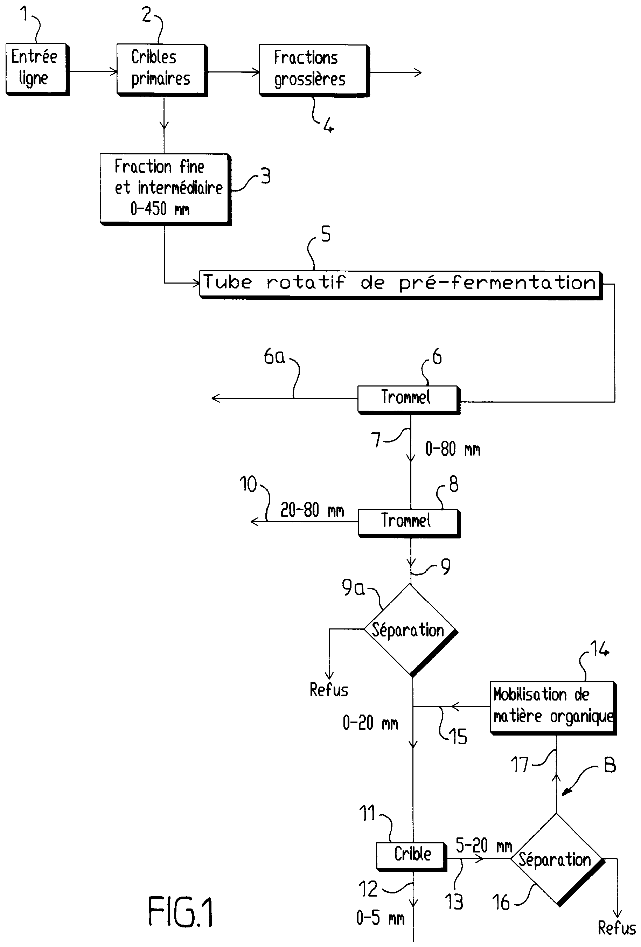

En se reportant à Fig. 1, on peut voir quelques étapes d'un procédé de traitement de déchets, en particulier d'ordures ménagères, selon l'invention. Les ordures ménagères peuvent contenir des matières organiques mélangées à des inertes, notamment métaux, matières minérales, plastiques, verre.

L'entrée des déchets dans la ligne de traitement correspond à l'étape 1. Les déchets sont ensuite introduits, lors de l'étape 2, dans un ou plusieurs cribles primaires qui permettent de séparer une fraction fine et intermédiaire 3 de granulométrie comprise, par exemple, entre 0 et 450 mm et préférablement entre 200 et 400 mm. Le refus sortant des cribles primaires 2 correspond aux fractions grossières 4 qui sont évacuées vers un traitement approprié.

La fraction 3 fine et intermédiaire est introduite dans un tube rotatif de pré-fermentation 5. Les matières sortant du tube 5 sont dirigées vers un tambour de tri 6, également appelé trommel, dont les mailles sont prévues pour laisser passer une fraction 7 de granulométrie 0-80 mm par exemple, tandis que le refus 6a est dirigé vers un traitement approprié.

La fraction 7 est introduite dans un autre trommel 8 à mailles plus étroites que le tambour 6. Le trommel 8 laisse passer une fraction 9 de granulométrie 0-20 mm par exemple, tandis que le refus 10 qui correspond à une granulométrie 20-80 mm dans l'exemple considéré, est dirigé vers un traitement approprié.

La fraction 9 sortant du trommel 8 peut être d'abord soumise à une séparation 9a des lourds (fraction lourde), notamment à l'aide d'une table à rebonds ou autre dispositif de séparation des lourds, avec évacuation du refus, La fraction 9 sortant de la séparation 9a est ensuite soumise à un criblage fin 11 laissant passer une fraction de taille inférieure à 10 mm, notamment une fraction de 0-5 mm, désignée par 12 sur le schéma.

Le refus 13 du criblage fin 11 contient encore de la matière organique et l'invention vise à récupérer une majeure partie de cette matière organique présente dans le refus 13.

Le refus 13 est envoyé, selon une boucle de recirculation B sur un dispositif 14 de traitement de mobilisation de la matière organique, par voie mécanique et/ou biologique et/ou chimique. Le dispositif 14 est prévu pour fragmenter la matière organique présente dans le refus tout en assurant un maintien de la taille des inertes. Le refus ainsi traité, sortant du dispositif 14 est recirculé par la branche 15 à l'entrée de l'étape de criblage fin 11.

Des effluents, notamment de l'eau, à température ambiante ou préalablement réchauffée, avec éventuellement des additifs chimiques ou biologiques, peuvent être introduits dans le dispositif 14 pour favoriser le traitement de mobilisation de la matière organique.

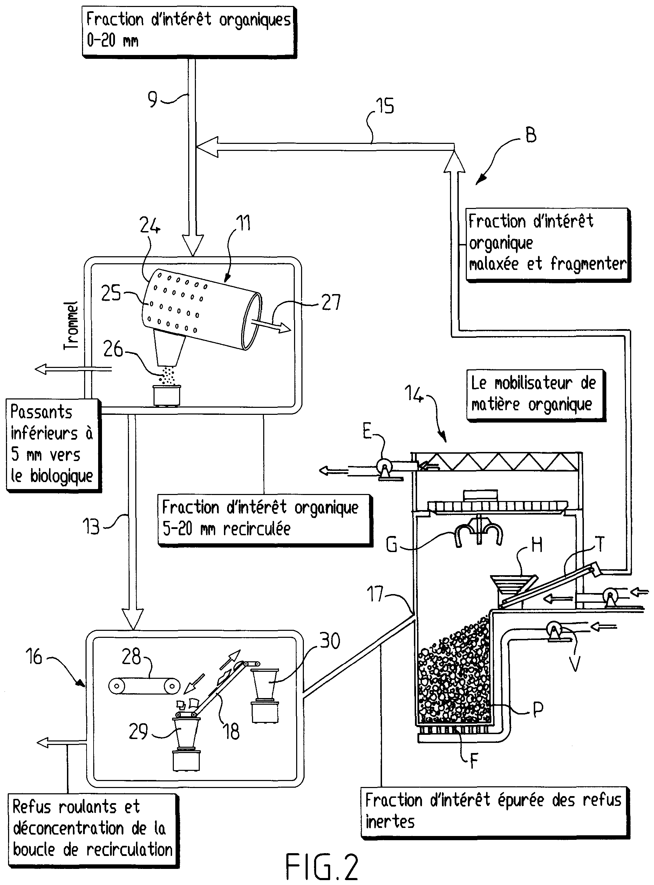

Une étape de séparation des inertes 16 est prévue entre la sortie du refus de criblage fin 13 et l'entrée 17 du traitement de mobilisation. Cette étape de séparation peut comprendre un tri balistique / densimétrique, et / ou un tri optique et / ou une séparation gravitaire sur tapis roulant incliné 18 comme illustré sur Fig. 2 et comme décrit plus loin.

L'étape 16 de séparation des inertes constitue également une étape de déconcentration des inertes relativement à la matière organique, en amont du traitement de mobilisation de cette matière organique.

Après l'étape 16 de décantation/séparation des inertes, la matière est introduite dans le dispositif de mobilisation 14 de la matière organique. Ce dispositif 14 comporte, selon l'exemple de réalisation de Fig. 2, illustré en coupe verticale schématique, un récipient de stockage aéré et brassé, notamment une fosse P. Le refus 13 de criblage séjourne dans la fosse P pendant un temps déterminé, au-dessus d'un plancher F muni d'ouvertures d'aération. Une chambre dans laquelle de l'air est injecté par une soufflante V est prévue sous le plancher F. Cet air est avantageusement réchauffé, de préférence avec des ressources thermiques générées par l'installation, notamment par l'air de refroidissement de moteurs. Un système de brassage de type pont-grappin G, par exemple, permet d'assurer un retournement et un désagrégement régulier du produit stocké afin d'en optimiser son aération et d'éviter les phénomènes de tassement et/ou d'agglomération. La partie supérieure du dispositif 14 comporte des ouvertures pour une évacuation canalisée, par ventilateur E, des gaz produits par les réactions biologiques conduisant à la réduction de la granulométrie de la fraction d'intérêt biologique.

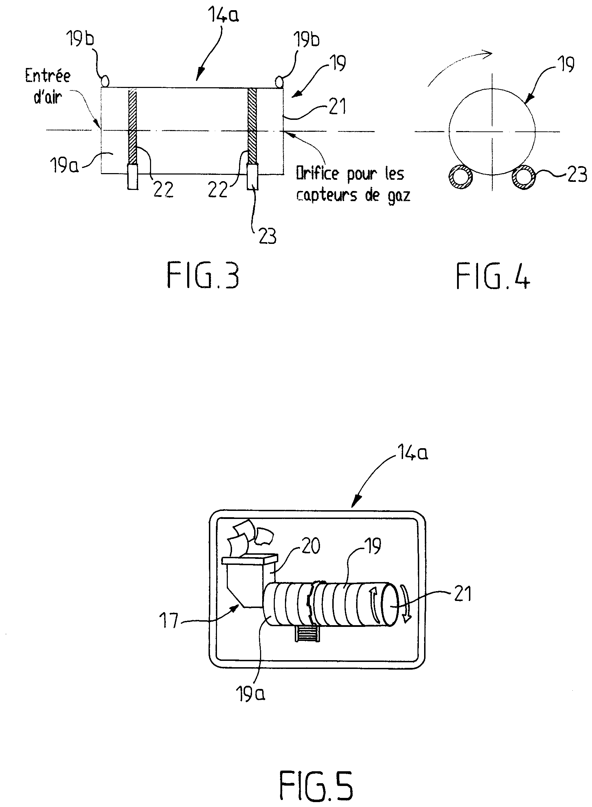

Le cas échéant, le traitement de mobilisation selon Fig. 2 peut être combiné en série avec la variante à tube rotatif des Fig. 3-5.

Selon ces Fig.3-5, le dispositif de mobilisation 14a des matières organiques comporte un tube rotatif 19 dont la paroi cylindrique est fermée. Le refus de criblage fin est introduit à une entrée 19a du tube 19, notamment à travers une goulotte 20 (Fig. 5), et est évacué à l'autre extrémité axiale 21.

Comme illustré sur Fig. 3, le tube 19 comporte, sur sa paroi cylindrique, deux chemins de roulement 22 espacés axialement propres à rouler sur des galets 23 (Fig. 4). De préférence, le tube 19 est aéré, éventuellement avec de l'air chaud, et comporte du côté de l'entrée 19a, un ou plusieurs orifices d'entrée d'air tandis que du côté de la sortie 21 des orifices peuvent être prévus pour capter les gaz provenant du tube 19.

Le tube rotatif 19 est avantageusement équipé de moyens, non visibles, propres à fragmenter la matière organique, en particulier de boulets ou de couteaux. Les moyens employés permettent de fragmenter la matière organique mais sont prévus pour éviter de fragmenter les inertes résiduels. Des moyens techniques à ultrasons, ou des additifs biologiques ou chimiques peuvent être utilisés pour la fragmentation de la matière organique dans le tube 19, en complément ou en remplacement de moyens mécaniques.

La vitesse périphérique sur la paroi du tube 19 peut être avantageusement variable, comprise entre 5 et 30 cm/s, préférablement 10 cm/s soit une vitesse de rotation du tube 19 de 1 à 8 tours par minute.

Fig. 2 représente plus en détail la boucle de recirculation B de Fig. 1. La sortie 9 du schéma de Fig. 1, correspondant à une fraction d'intérêt organique 0-20 mm est introduite à l'entrée du crible 11. Ce crible peut être de type vibrant ou de type trommel ; le présent exemple décrit un trommel 24 d'axe géométrique incliné. Le trommel 24 présente des orifices 25 de 5 mm de diamètre. Les matières qui traversent les orifices 25 constituent des passants 26 de granulométrie inférieure à 5 mm et sont transférées vers le traitement biologique.

La fraction 27 de granulométrie plus forte, supérieure à 5 mm, constitue une fraction d'intérêt organique 5-20 mm qui constitue le refus 13 dirigé vers l'étape de séparation et de déconcentration des matières recirculées.

L'étape 16 de déconcentration est assurée par un dispositif comportant un tapis roulant horizontal 28 qui reçoit le refus 13 et qui le déverse en partie inférieure du tapis roulant incliné 18, lequel peut être soumis à des vibrations. Les matières inertes se retrouvent en partie basse du tapis roulant 18 et sont récupérées par une trémie 29 pour être évacuées sous forme de refus roulants résultant de la déconcentration des matières de la boucle de recirculation B.

La fraction d'intérêt organique, épurée des refus inertes, est récupérée à l'extrémité supérieure du tapis 18 dans une trémie 30 et est dirigée vers l'entrée 17 du dispositif 14 de mobilisation de la matière organique.

Le pont-grappin G sert également de moyen de reprise ou de transfert de la fraction organique malaxée et fragmentée qui est déversée dans une trémie H puis sur un convoyeur à bande T. La fraction d'intérêt organique malaxée et fragmentée provenant du convoyeur T est ensuite dirigée par une branche 15 de recirculation vers la fraction 9 et l'entrée du crible 11.

Le dispositif 14 constitue un mobilisateur de matières organiques qui permet d'accroître le taux de matières organiques de fraction fine inférieure à la maille du criblage choisi qui, dans l'exemple représenté, est de 5 mm. Il en est de même pour la variante 14a du dispositif avec tube rotatif 19.

Le dispositif mobilisateur 14, 14a peut aussi être appliqué en sortie d'un crible de maille supérieure à celle envisagée de 5 mm.

Sur la boucle de recirculation B peuvent être positionnés des dispositifs de tri optique, de table à rebonds, de crible supplémentaire, et/ou de table densimétrique.

### Exemple de réalisation

Cet exemple concerne une réalisation pour traiter des ordures ménagères de fractions ciblées 20-0 mm, constituant la fraction d'intérêt, provenant d'une succession de séparations mécaniques et biologiques.

Des valorisations matières sont également mises en oeuvre. Les fractions de granulométrie supérieures à 80 mm et comprises entre 20 et 80 mm sont dirigées de préférence vers des étapes de valorisation des matières plastiques, des ferreux et non-ferreux. Les papiers, les cartons sont aussi réinjectés dans la filière de traitement.

Un temps de séjour moyen dans le dispositif 14 de mobilisation de la matière organique est compris entre quelques heures et quelques jours, de préférence 24 h environ. Ce paramètre du processus dépend de la qualité de la matière entrante.

La mise en oeuvre du procédé de l'invention a permis d'établir un transfert de la matière organique fermentescible de la fraction 5-20 mm vers la fraction 0-5 mm.

La réalisation expérimentale a permis de constater une extraction d'environ 20 à 70 % de la matière organique comprise dans la fraction 5-20 mm, suite au passage dans le mobilisateur 14 de matière organique.

Les paramètres de fonctionnement testés ont été les suivants :

### effets mécaniques

- taux de remplissage en volume du tube rotatif 19 : 75 % et 60 %
- vitesse de rotation du tube 19 : 1 tour/min et 3 tours/min

### effets biologiques

- aération : 10 m³ d'air/m³ d'ordures ménagères/h (heure)
   5 m³ d'air/m³ d'ordures ménagères/h
   3 m³ d'air/m³ d'ordures ménagères/h
- humidité des matières : 50 à 60 %
- durée de traitement : 24 h à 72h

### Applications industrielles

L'étape de recirculation des fractions fermentescibles fines, selon l'invention, permet d'optimiser le bilan matière :
- dans la filière méthanisation/compostage : en produisant davantage de biogaz ramené au tonnage effectivement traité, l'augmentation étant d'environ 15 à 35 % en plus. La fraction à méthaniser est réduite, ce qui permet de réduire les dépenses d'investissement, tandis que la concentration en méthane du biogaz est majorée entre 2 et 8 %, généralement d'environ 3 %.
- dans la filière compostage seul, ou méthanisation/compostage, la production des composts normés est de bonne qualité, et augmentée d'environ 30 % par rapport à une filière de l'état de la technique. Le bilan global est d'environ 25 à 35 % de compost/entrants, comparé à 20 à 25 % de compost/entrants selon une filière de l'état de la technique.

Toujours selon l'exemple envisagé d'un pré-tri ciblé à 20 mm au niveau du trommel 8 de Fig. 1, et d'un seuil de coupure 5 mm pour le crible 11, les différentes possibilités sont les suivantes :
***A-*** *Dans le cas d'une méthanisation,* la matière de granulométrie inférieure à 5 mm est reprise et traitée en digestion dans un appareil digesteur. Ce digesteur produit un digestat qui est ensuite traité en compostage.

Le digestat est envoyé dans une mélangeuse dans laquelle est introduit un élément structurant, par exemple des déchets de bois ou des déchets verts, et/ou du refus d'affinage d'une granulométrie supérieure à 20 mm recirculé. La matière sortant de la mélangeuse est soumise à une étape de maturation puis à une étape d'affinage pour une granulométrie inférieure à 20 mm qui va constituer la matière pour le compost. Le refus d'affinage, de granulométrie supérieure à 20 mm, est recirculé sous forme de structurant vers la mélangeuse.

### B- Cas du compostage direct sans méthanisation

Dans ce cas, la fraction 0-5 mm provenant du crible 11 de Fig. 1 est introduite directement dans la mélangeuse évoquée précédemment et est soumise aux mêmes étapes avec boucle de recirculation pour le refus d'affinage supérieur à 20 mm. La fraction sortant de l'affinage, de granulométrie inférieure à 20 mm, va servir au compost.

Le procédé et l'installation de l'invention permettent de mobiliser la matière organique dans la fraction fine située entre 80 et 0 mm, avantageusement entre 30 et 0 mm, et de préférence entre 20 et 5 mm. La mobilisation des matières organiques par le dispositif 14 ou 14a permet d'accroître le taux de matières organiques de fraction fine inférieure à la maille du criblage fin 11 choisie.

## Revendications

1. Procédé de traitement de déchets, en particulier d'ordures ménagères, contenant des matières organiques mélangées à des inertes, notamment métaux, matières minérales, plastiques, verre, procédé du genre de ceux selon lesquels :
- les déchets sont soumis à un premier criblage (2),
- la fraction (3) des déchets ayant traversé ce premier criblage est soumise à un traitement de pré-fermentation dans un tube rotatif (5) avec alimentation à une extrémité et extraction à l'autre extrémité,
- les déchets, après traitement de pré-fermentation et, le cas échéant après criblage intermédiaire, sont soumis à une étape de criblage fin (11) pour ne laisser passer qu'une fraction de matières de taille inférieure ou égale à une dimension M, en particulier inférieure ou égale à 20 mm, correspondant essentiellement à des matières organiques, et pour donner un refus de criblage fin,
- le refus (13) de criblage fin est soumis à un traitement (14, 14a) mécanique et/ou biologique et/ou chimique pour la mobilisation de la matière organique, ce traitement étant suffisant pour fragmenter la matière organique présente dans le refus de criblage fin tout en assurant un maintien de la taille des inertes et en évitant leur fragmentation,
**caractérisé en ce que** :
- le refus de criblage fin, après ce traitement, est recirculé (15) vers l'étape de criblage fin (11).

2. Procédé selon la revendication 1, **caractérisé en ce qu'**une étape (16) de déconcentration/séparation des inertes est prévue entre la sortie du refus de criblage fin et l'entrée du traitement de mobilisation, pour écarter les inertes de la fraction organique.

3. Procédé selon la revendication 2, **caractérisé en ce que** l'étape de déconcentration/séparation comprend un tri balistique / densimétrique, et / ou un tri optique et / ou une séparation gravitaire sur tapis roulant incliné (18).

4. Procédé selon l'une quelconque des revendications précédentes, **caractérisé en ce que** le criblage fin est effectué avec un maillage dont la dimension M est comprise entre 5 et 10 mm, tandis que les déchets soumis à ce criblage fin sont issus d'un crible situé en amont dont la maille est de 20 mm.

5. Procédé selon l'une quelconque des revendications 1 à 4, **caractérisé en ce que** le traitement de mobilisation de matières organiques comprend une étape de stockage (14, 14a) dans un dispositif de stockage aéré et brassé, en particulier une fosse (P), pendant une durée de quelques heures à plusieurs jours, en particulier de 24 h.

6. Procédé selon l'une quelconque des revendications précédentes, **caractérisé en ce que** le traitement de mobilisation des matières organiques comprend le passage des matières dans un tube rotatif (19), équipé de moyens propres à fragmenter la matière organique, sans réduire la taille des inertes, en particulier de boulets ou de couteaux.

7. Procédé selon la revendication 6, **caractérisé en ce que** le tube rotatif (19) est aéré avec de l'air à température ambiante ou réchauffé.

8. Installation pour la mise en oeuvre d'un procédé selon la revendication 1, comprenant :
- un premier crible (2) pour les déchets,
- un tube rotatif (5) de pré-fermentation avec alimentation à une extrémité par la fraction de déchets ayant traversé le premier crible, et extraction à l'autre extrémité,
- des cribles intermédiaires éventuels,
- et un crible fin (11) pour laisser passer une fraction de matières de taille inférieure ou égale à une dimension M, en particulier inférieure ou égale à 20 mm,
- un dispositif (14, 14a) à action mécanique et/ou biologique et/ou chimique de mobilisation des matières organiques du refus de criblage fin, propre à fragmenter la matière organique présente dans le refus tout en assurant le maintien de la taille des inertes,
**caractérisée en ce qu'**elle comporte :
- et une boucle de recirculation (B) du refus de criblage fin avec passage par ledit dispositif de mobilisation (14, 14a) de matières organiques vers l'étape de criblage fin (11).

9. Installation selon la revendication 8, **caractérisée en ce qu'**elle comporte un équipement (16) de déconcentration/séparation des inertes prévu entre la sortie du refus de criblage fin et l'entrée du dispositif de mobilisation (14, 14a), pour écarter les inertes de la fraction organique.

10. Installation selon la revendication 9, **caractérisée en ce que** l'équipement de déconcentration/séparation comprend un tri balistique / densimétrique, et / ou un tri optique et / ou une séparation gravitaire sur tapis roulant incliné (18).

11. Installation selon l'une des revendications 8 à 10, **caractérisée en ce que** le criblage fin est effectué avec un maillage dont la dimension M est égale à 5 mm, tandis que les déchets soumis à ce criblage fin sont issus d'un crible situé en amont dont la maille est de 20 mm.

12. Installation selon l'une des revendications 8 à 11, **caractérisée en ce que** le dispositif de mobilisation des matières organiques comprend un dispositif de stockage (14, 14a) aéré et brassé avec de l'air à température ambiante ou réchauffé.

13. Installation selon la revendication 12, **caractérisée en ce que** le dispositif de stockage aéré et brassé (14) comprend une fosse (P) avec plancher muni d'ouvertures d'aération.

14. Installation selon l'une des revendications 8 à 12, **caractérisée en ce que** le dispositif de mobilisation (14a) des matières organiques comprend un tube rotatif (19), équipé de moyens propres à fragmenter la matière organique, sans réduire la taille des inertes, en particulier de boulets ou de couteaux.

## Patentansprüche

1. Verfahren zur Behandlung von Abfällen, insbesondere von Haushaltsabfällen, welche organische Stoffe enthalten, die mit inerten Stoffen gemischt sind, insbesondere Metalle, Mineralstoffe, Kunststoffe, Glas, wobei das Verfahren von dem Typ ist, bei dem:
- die Abfälle einer ersten Siebung (2) unterzogen werden,
- der Anteil (3) der Abfälle, der diese erste Siebung durchlaufen hat, einer Vorgärung in einem drehbaren Rohr (5) unterzogen wird, das auf einer Seite einen Einlauf und auf der anderen Seite einen Auslauf aufweist,
- die Abfälle nach der Vorgärung und gegebenenfalls nach einer Zwischensiebung einem Feinsiebungsschritt (11) unterzogen werden, um lediglich eine Fraktion von Stoffen mit einer Teilchengröße von kleiner oder gleich einer Größe M, insbesondere kleiner oder gleich 20 mm passieren zu lassen, welche im Wesentlichen den organischen Stoffen entspricht, und um einen Siebüberlauf zu erzeugen,
- der Überlauf (13) der Feinsiebung einer mechanischen und/oder biologischen und/oder chemischen Behandlung (14, 14a) zur Mobilisierung der organischen Stoffe unterzogen wird, wobei diese Behandlung ausreichend ist, um die im Überlauf der Feinsiebung enthaltenen organischen Stoffe zu fragmentieren und gleichzeitig eine Bewahrung der Größe der inerten Stoffe sicherzustellen und ihre Fragmentierung zu vermeiden,
**dadurch gekennzeichnet, dass**
- nach dieser Behandlung der Überlauf der Feinsiebung in den Feinsiebungsschritt (11) zurückgeführt wird (15) .

2. Verfahren gemäss Anspruch 1, **dadurch gekennzeichnet, dass** ein Dekonzentrier-/Trennungsschritt (16) für die inerten Stoffe zwischen dem Ausgang des Überlaufs der Feinsiebung und dem Eingang der Mobilisierungsbehandlung vorgesehen ist, um die inerten Stoffe von der organischen Fraktion abzutrennen.

3. Verfahren gemäss Anspruch 2, **dadurch gekennzeichnet, dass** der Dekonzentrier-/Trennungsschritt eine ballistische / densitometrische Sortierung und/oder eine optische Sortierung und/oder eine Schwerkrafttrennung auf einem schrägen Förderband (18) umfasst.

4. Verfahren gemäss einem der vorhergehenden Ansprüche, **dadurch gekennzeichnet, dass** die Feinsiebung mit einer Maschenweite M zwischen 5 und 10 mm durchgeführt wird, wobei die dieser Feinsiebung unterzogenen Abfälle aus einem sich stromaufwärts befindlichen Sieb stammen, dessen Maschenweite 20 mm beträgt.

5. Verfahren gemäss einem der Ansprüche 1 bis 4, **dadurch gekennzeichnet, dass** die Mobilisierungsbehandlung der organischen Stoffe einen Speicherschritt (14, 14a) in einer belüfteten und umgewälzten Speichervorrichtung, insbesondere eine Grube (P), während einer Dauer von mehreren Stunden bis mehreren Tagen, insbesondere von 24 h, umfasst.

6. Verfahren gemäss einem der vorhergehenden Ansprüche, **dadurch gekennzeichnet, dass** die Mobilisierungsbehandlung der organischen Stoffe den Durchfluss der Stoffe durch ein drehbares Rohr (19) umfasst, das mit Mitteln zur Fragmentierung der organischen Stoffe ausgestattet ist, insbesondere mit Kugeln oder Messern, ohne die Größe der inerten Stoffe zu reduzieren.

7. Verfahren gemäss Anspruch 6, **dadurch gekennzeichnet, dass** das drehbare Rohr (19) mit Luft bei Umgebungstemperatur oder mit erwärmter Luft belüftet wird.

8. Anlage zur Durchführung eines Verfahrens gemäss Anspruch 1, bestehend aus:
- einem ersten Sieb (2) für die Abfälle,
- einem drehbaren Rohr (5) zur Vorgärung der Abfallfraktion, die das erste Sieb durchlaufen hat, mit einem Einlauf auf einer Seite und einem Auslauf auf der anderen Seite,
- gegebenenfalls Zwischensieben,
- und einem Feinsieb (11), um eine Fraktion von Stoffen mit einer Teilchengröße von kleiner oder gleich einer Größe M, insbesondere kleiner oder gleich 20 mm passieren zu lassen,
- einer Vorrichtung (14, 14a) mit einer mechanischen und/oder biologischen und/oder chemischen Wirkung zur Mobilisierung der organischen Stoffe aus dem Überlauf der Feinsiebung, die zur Fragmentierung der im Überlauf enthaltenen organischen Stoffe geeignet ist und gleichzeitig die Bewahrung der Größe der inerten Stoffe sicherstellt,
**dadurch gekennzeichnet, dass** sie
- des Weiteren eine Rückführschleife (B) für den Überlauf der Feinsiebung aufweist, in der die organischen Stoffe durch die Mobilisierungsvorrichtung (14, 14a) zu dem Feinsiebungsschritt (11) geführt werden.

9. Anlage gemäss Anspruch 8, **dadurch gekennzeichnet, dass** sie eine Dekonzentrier-/Trennungsvorrichtung (16) für die inerten Stoffe aufweist, die zwischen dem Ausgang des Überlaufs der Feinsiebung und dem Eingang der Mobilisierungsvorrichtung (14, 14a) vorgesehen ist, um die inerten Stoffe von der organischen Fraktion abzutrennen.

10. Anlage gemäss Anspruch 9, **dadurch gekennzeichnet, dass** die Dekonzentrier-/Trennungsvorrichtung eine ballistische / densitometrische Sortierung und/oder eine optische Sortierung und/oder eine Schwerkrafttrennung auf einem schrägen Förderband (18) umfasst.

11. Anlage gemäss einem der Ansprüche 8 bis 10, **dadurch gekennzeichnet, dass** die Feinsiebung mit einer Maschenweite M von 5 mm durchgeführt wird, wobei die der Feinsiebung unterzogenen Abfälle aus einem sich stromaufwärts befindlichen Sieb stammen, dessen Maschenweite 20 mm beträgt.

12. Anlage gemäss einem der Ansprüche 8 bis 11, **dadurch gekennzeichnet, dass** die Mobilisierungsvorrichtung für die organischen Stoffe eine mit Luft bei Umgebungstemperatur oder mit erwärmter Luft belüftete und umgewälzte Speichervorrichtung (14, 14a) umfasst.

13. Anlage gemäss Anspruch 12, **dadurch gekennzeichnet, dass** die belüftete und umgewälzte Speichervorrichtung (14) eine Grube (P) mit einem Boden umfasst, welcher mit Belüftungsöffnungen versehen ist.

14. Anlage gemäss einem der Ansprüche 8 bis 12, **dadurch gekennzeichnet, dass** die Mobilisierungsvorrichtung (14a) für die organischen Stoffe ein drehbares Rohr (19) umfasst, das mit Mitteln zur Fragmentierung der organischen Stoffe ausgestattet ist, insbesondere mit Kugeln oder Messern, ohne die Größe der inerten Stoffe zu reduzieren.

## Claims

1. A method for treating waste, in particular municipal waste, containing organic matters mixed with inerts, in particular metals, mineral matters, plastics, glass, a method of the kind of those according to which:
- the waste is subjected to a first screening (2),
- the fraction (3) of the waste that passed through this first screening is subjected to a pre-fermentation treatment in a rotary tube (5) with feeding at one end and extraction at the other end,
- the waste, after pre-fermentation treatment and, possibly after intermediate screening, is subjected to a fine screening step (11) to pass only a fraction of matters with a size lower than or equal to a dimension M, in particular lower than or equal to 20 mm, essentially corresponding to organic matters, and to give a fine screen overflow,
- the fine screen overflow (13) is subjected to a mechanical and/or biological and/or chemical treatment (14, 14a) for mobilisation of the organic matter, this treatment being sufficient to fragment the organic matter present in the fine screen overflow while ensuring maintaining of the size of inerts and avoiding their fragmentation,
**characterised in that**:
- the fine screen overflow, after this treatment, is recirculated (15) to the fine screening step (11).

2. The method according to claim 1, **characterised in that** a step (16) of deconcentrating/separating the inerts is provided between the outlet of the fine screen overflow and the inlet of the mobilisation treatment, to discard the inerts from the organic fraction.

3. The method according to claim 2, **characterised in that** the deconcentration/separation step comprises a ballistic/densimetric sorting, and/or an optical sorting and/or a gravity separation on an inclined conveyor (18).

4. The method according to any of the preceding claims, **characterised in that** the fine screening is made with a meshing the dimension M of which is between 5 and 10 mm, whereas the waste subjected to this fine screening comes from an upstream screen the mesh of which is 20 mm.

5. The method according to any of claims 1 to 4, **characterised in that** the organic matter mobilisation treatment comprises a storage step (14, 14a) in an aerated and agitated storage device, in particular a bunker (P), for a duration of a few hours to several days, in particular 24 h.

6. The method according to any of the preceding claims, **characterised in that** the organic matter mobilisation treatment comprises passing matters in a rotary tube (19), equipped with means able to fragment the organic matter, without reducing the size of the inerts, in particular balls or knives.

7. The method according to claim 6, **characterised in that** the rotary tube (19) is aerated with warmed air or air at ambient temperature.

8. A facility for implementing a method according to claim 1, comprising:
- a first screen (2) for waste,
- a pre-fermentation rotary tube (5) with feeding at one end with the fraction of waste that passed through the first screen, and extraction at the other end,
- possible intermediate screens,
- and a fine screen (11) to pass a fraction of matters with a size lower than or equal to a dimension M, in particular lower than or equal to 20 mm,
- a mechanical and/or biological and/or chemical action device (14, 14a) for mobilisation of the organic matters of the fine screen overflow, able to fragment the organic matter present in the overflow while ensuring maintaining of the size of the inerts,
**characterised in that** it includes:
- and a recirculation loop (B) of the fine screen overflow with passing organic matters through said mobilisation device (14, 14a) to the fine screening step (11).

9. The facility according to claim 8, **characterised in that** it includes an equipment (16) of deconcentrating/separating the inerts provided between the outlet of the fine screen overflow and the inlet of the mobilisation device (14, 14a), to discard the inerts from the organic fraction.

10. The facility according to claim 9, **characterised in that** the deconcentration/separation equipment comprises a ballistic/densimetric sorting, and/or an optical sorting and/or a gravity separation on an inclined conveyor (18).

11. The facility according to one of claims 8 to 10, **characterised in that** the fine screening is made with a meshing the dimension M of which is equal to 5 mm, whereas the waste subjected to this fine screening comes from an upstream screen the mesh of which is 20 mm.

12. The facility according to one of claims 8 to 11, **characterised in that** the organic matter mobilisation device comprises a storage device (14, 14a) aerated and agitated with warmed air or air at ambient temperature.

13. The facility according to claim 12, **characterised in that** the aerated and agitated storage device (14) comprises a bunker (P) with a floor provided with aeration apertures.

14. The facility according to one of claims 8 to 12, **characterised in that** the organic matter mobilisation device (14a) comprises a rotary tube (19), equipped with means able to fragment the organic matter, without reducing the size of the inerts, in particular balls or knives.
